(19) **Europäisches Patentamt**

**European Patent Office**

**Office européen des brevets**

(11) **EP 0 752 391 B1**

(12) **EUROPEAN PATENT SPECIFICATION**

(45) Date of publication and mention
of the grant of the patent:
**17.03.2004 Bulletin 2004/12**

(51) Int Cl.⁷: $C02F\ 1/46$, C02F 1/469

(21) Application number: **96304974.7**

(22) Date of filing: **05.07.1996**

(54) **Production method of water for medical treatment**

Methode zur Herstellung von Medizinalwasser

Production d'eau médicinale

(84) Designated Contracting States:
**DE FR GB**

(30) Priority: **07.07.1995 JP 17190295**
**17.06.1996 JP 15564796**

(43) Date of publication of application:
**08.01.1997 Bulletin 1997/02**

(73) Proprietor: **Nihon Trim Co. Limited
Osaka-Shi, Osaka (JP)**

(72) Inventor: **Morisawa, Shinkatsu
Yodogawa-ku, Osaka-shi, Osaka (JP)**

(74) Representative: **Lipscombe, Martin John
Keith W Nash & Co,
Pearl Assurance House,
90-92 Regent Street
Cambridge CB2 1DP (GB)**

(56) References cited:
EP-A- 0 070 346          WO-A-89/02709
WO-A-95/02559          GB-A- 1 512 146
US-A- 4 122 010          US-A- 4 834 888

• DATABASE WPI Section Ch, Week 9631 Derwent
Publications Ltd., London, GB; Class B06, AN
96-303568 XP002034864 & JP 07 185 550 A
(HAYASHI H) , 25 July 1995
• DATABASE WPI Section Ch, Week 9329 Derwent
Publications Ltd., London, GB; Class D15, AN
93-234440 XP002033457 & SU 1 752 315 A
(MOSKOVSKOE ASSEMBLY MANAGEMENT) , 7
August 1992
• DATABASE WPI Section Ch, Week 9522 Derwent
Publications Ltd., London, GB; Class D15, AN
95-166591 XP002034833 & JP 07 088 475 A
(MATSUO SEITAI BUTSURI KENKYUSHO YG) , 4
April 1995
• DATABASE WPI Section Ch, Week 9433 Derwent
Publications Ltd., London, GB; Class D15, AN
94-266779 XP002033456 & JP 06 193 971 A
(MITSUBISHI ELECTRIC CORP) , 15 July 1994

**Description**

BACKGROUND OF THE INVENTION

Field of the Invention

The present invention generally relates to water for medical treatment, and more particularly, to a method of producing water for medical treatment that has potency to abolish Superoxide Anion Radical: $O_2^-\cdot$ (referred to as "SAR" hereinafter), generated in a body.

Description of the Background Art

Significant advance has been seen in the field of modern medicine, and the variety and number of new medicines and drugs available on the market is increasing. Development in medicomechanical instrumentation has allowed a more accurate grasp of the human body, so that a more appropriate treatment can be applied. Many people graduate from medical colleges every year to practice medicine, and the technique in medical treatment is in progress.
However, the number of people with disorders is increasing in spite of such progress in medical science. Accordingly, the total amount of medical fee shows no decrease. It is considered that this is due to the fact that the symptom of the disease is treated nosotropically, and that fundamental therapy is not applied.
Recently, it has been confirmed that the SAR generated within the human body is the trigger of various disease and illness. It is considered that SAR is generated as a result of the oxygen in blood being subjected to the effect of ischemic re-perfusion, intracorporeal bacteria, uric acid in blood, fat, and sugar to be reduced or by the influence of neutrophils.
There is a great amount of SAR in the blood of individuals with abundant uric acid in the blood, fat, and sugar or with disease. SAR reacts with DNA and the like in the body to induce symptoms and disease such as allergic dermatitis.
A process or method of abolishing this SAR from blood is now drawing the attention of doctors and people working at medical institutes all over the world.
Conventionally, β-carotene, vitamin C, vitamin E and SOD (Super Oxide•Dismutase) food are typically known to abolish SAR. However, there is a problem that excessive ingestion thereof will cause reaction such as oxidation effect.
SU 1752 315 (Database WPI Week 9329 Derwent Publications Ltd, London, AN 93-234440 XP 002033457) discloses drinking water for broilers, the water having a redox potential in the range -50 - -400mV, but does not disclose details of the method by which the water is produced.
JP 07 088 475 (Database WPI Week 9522 Derwent Publications Ltd, London, AN 95-166591 XP 002034833) discloses water having a redox potential in the range -50 - -650mV, prepared by electrolysis of a solution of sodium or potassium chloride.
JP 06 193 971 (Database WPI Week 9433 Derwent Publications Ltd, London, AN 95-166591 XP 002033456) discloses water with reduced redox potential prepared by electrolytic treatment of tap water but does not disclose details of the method of its preparation.
GB 1 512 146 discloses apparatus for removing microbial contaminants from water. WO 95/02559 relates to apparatus for regenerating liquids, such as dialysis liquids. US 4122010 discloses apparatus for treating blood: there is no eletrolytic step involved.

SUMMARY OF THE INVENTION

In view of the foregoing, an object of the present invention is to provide a method of producing water for medical treatment that has potency to abolish SAR that becomes the trigger of various disease.
According to an aspect of the present invention, water for medical treatment is produced by the method defined in claim 1, and has an oxidation-reduction potential value within the range of -150 mV $\sim$ 0 mV measured using a platinum electrode. Sterile water having an oxidation-reduction potential measured value of -68 mV $\sim$ +55 mV is obtained by boiling the water.
By electrolyzing water under particular conditions set forth in the following, water is generated at the cathode side (referred to as cathode water hereinafter). The inventors found that the obtained cathode water is rich in electron ($e^-$) and proton ($H^+$) with potency of abolishing SAR generated within the body.
The reaction mechanism of the cathode water for removing SAR is as follows.

$$O_2^- \text{ (Superoxide Anion Radical)} + e^- + 2H^+ \rightarrow H_2O_2 \text{ (hydrogen peroxide)}$$

$$H_2O_2 + e^- \rightarrow HO\bullet + HO^-$$

$$HO\bullet + e^- + H^+ \rightarrow H_2O$$

More specifically, it is believed that SAR which is the cause of various disease is combined with electron (e⁻) and proton (H⁺) included in the cathode water to be reduced and converted into $H_2O$ (water) such as to be abolished.

The method of the invention for producing water for medical treatment typically includes the use of an electrolytic water generator. The electrolytic water generator includes a cathode chamber with a cathode and an anode chamber with an anode separated by a diaphragm. Raw water including at least sodium, potassium, magesium, and calcium ions are supplied to respective anode and cathode chambers. A current within the range of 0.16 mA/cm$^2$ ∼ 3.22 mA/cm$^2$ per two electrodes and one diaphragm is applied for at least 0.5 seconds and not more than 5 seconds across the cathode and the anode to electrolyze the raw water. Then, the water within the cathode chamber is extracted. It was found that the cathode water obtained by such a method has the potency to remove SAR.

Conveniently dialysis apparatus includes an electrolytic water generation apparatus for electrolyzing water, a filter apparatus for filtering cathode water fed from an electrolytic liquid reservoir tank, and a dialysis liquid supply apparatus for supplying the cathode water introduced from the filter apparatus as dialysis liquid.

Since the dialysis apparatus includes an electrolytic water generation apparatus, cathode water that can remove SAR that becomes the cause of disease can be supplied to the human body as dialysis liquid. Therefore, SAR in the blood of a patient can be removed in addition to the effect of hemodialysis.

Desirably the dialysis apparatus includes an electrolytic water generation apparatus for electrolyzing water, a boiling unit for boiling cathode water fed from the electrolytic water generation apparatus, and a dialysis liquid supply apparatus for supplying cathode water introduced from the boiling apparatus as dialysis liquid.

Since the dialysis apparatus includes a boiling apparatus, the bacteria in the cathode water can be disinfected.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a diagram showing the concept of an electrolytic water generator suitable for producing water for medical treatment according to the method of the present invention.

Fig. 2 is a diagram showing the result of potency of abolishing SAR from the water for medical treatment produced by the first embodiment using blood of a 72-year-old male patient.

Fig. 3 shows the result of potency of abolishing SAR from the water for medical treatment produced by the first embodiment using the blood of a 38-year-old male patient.

Fig. 4 shows the result of the effect of water produced by the method of the present invention using the blood of a patient suffering from acute pancreatitis.

Fig. 5 is a diagram showing the concept of a conventional dialysis apparatus for describing a dialysis apparatus.

Fig. 6 is a diagram showing the concept of the dialysis apparatus using the method of the invention.

Fig. 7 shows the characterizing portion of the dialysis apparatus in detail.

Fig. 8 is a diagram showing the concept of a dialysis apparatus using the method of the present invention.

DESCRIPTION OF THE PREFERRED EMBODIMENTS

First Embodiment

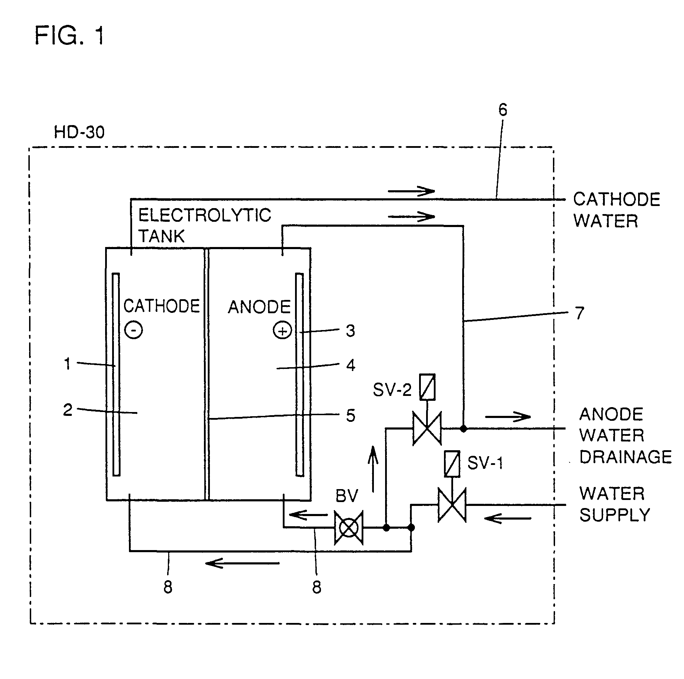

Referring to Fig. 1, an electrolytic water generator (HD-30) suitable for use in the method of the invention includes a cathode chamber 2 with a cathode 1 and an anode chamber 4 with an anode 3. Cathode chamber 2 is separated from anode chamber 4 by a diaphragm 5. A cathode liquid outlet pipe 6 from which cathode liquid is drawn out is connected to cathode chamber 2. A drain pipe 7 for discharging anode water outward is connected to anode chamber 4. A feed pipe 8 is connected to respective cathode and anode chambers 2 and 4 so that raw water including at least sodium, potassium, magnesium, and calcium such as tap water, ground water, and water from a well is supplied.

Raw water such as tap water, ground water, and well water is supplied to cathode chamber 2 and anode chamber 4. A current within the range of 0.16 mA/cm$^2$ ∼ 3.2 mA/cm$^2$ per two electrodes and one diaphragm is applied across cathode electrode 1 and anode electrode 3 for at least 0.5 seconds and not more than 5 seconds at room temperature (18°C ~ 22°C) to electrolyze the raw water. As a result, water for medical treatment which is the cathode water is obtained having the following properties. The oxidation-reduction potential (ORP) indicated in the present specification is measured using a platinum electrode by an ORP measuring apparatus (RM-12P) of Toa Denpa Co. Ltd.

PH8.0 ~ 9.5

ORP (oxidation-Reduction Potential): -150mV ~ 0mV

Cathode water of various characteristics is produced to study the potency of abolishing SAR from blood.

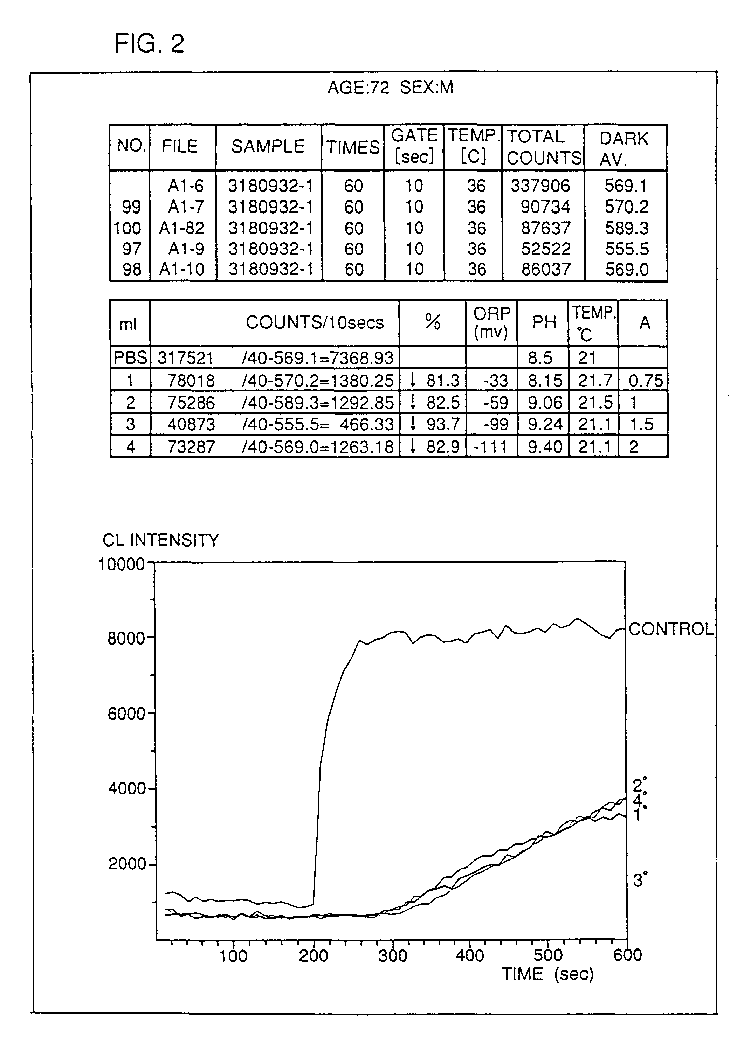

The graph of Fig. 2 indicates the amount of SAR in a mixture of blood extracted from a 72-year-old male patient and the above-described cathode water over time. In the graph of Fig. 2, time is plotted along the abscissa, and the amount of SAR is plotted along the ordinate. The curved line labeled "control" at the right side of the graph shows data when the above-described cathode water is not added. The curved lines labeled 1°, 2°, 3°, and 4° at the right side of the graph indicate data corresponding to the PBS test number.

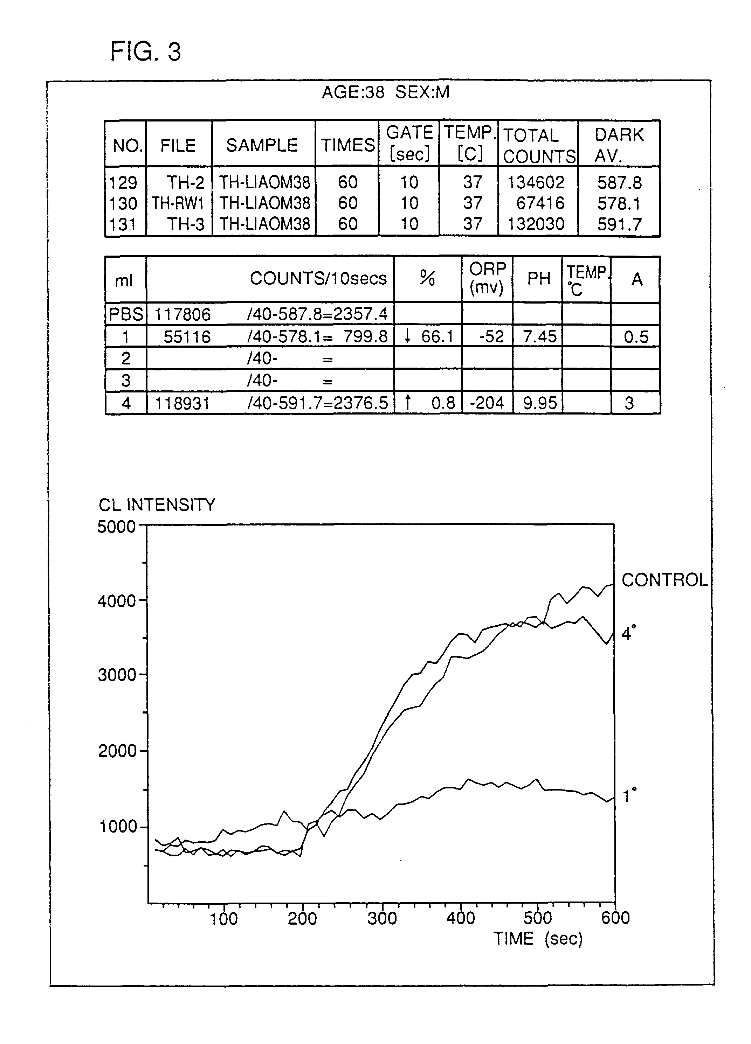

Fig. 3 shows the result of a similar test using blood extracted from a 38-year-old male patient.

Referring to Figs. 2 and 3, it was found that cathode water having an oxidation-reduction potential (ORP) of - 150 mV ~ 0 mV has the greatest potency to remove SAR.

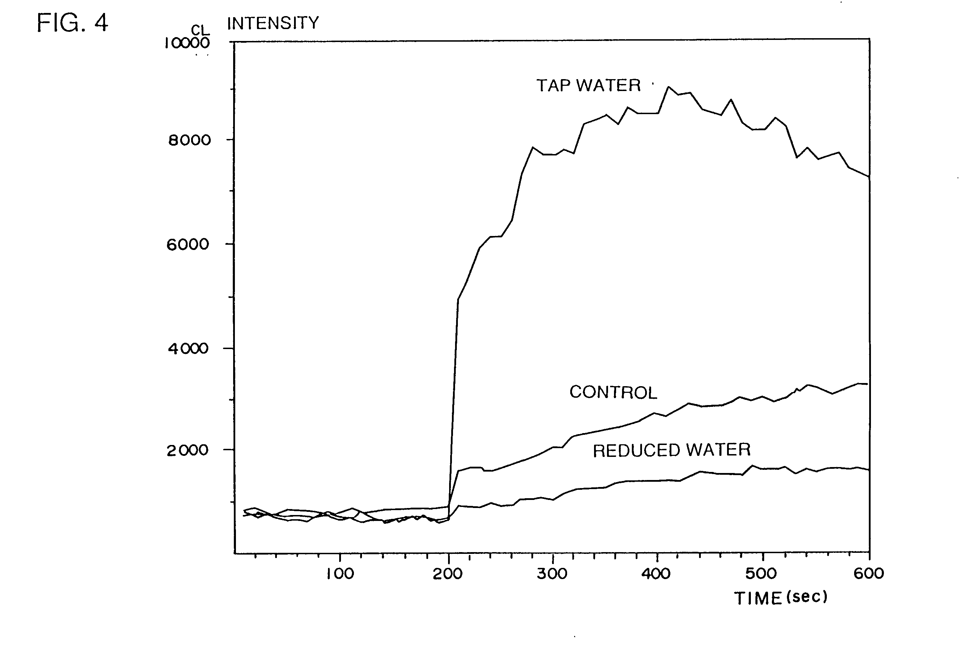

Fig. 4 shows the result of tracing the amount of SAR over time from a mixture of cathode water according to the present invention and blood extracted from a patient suffering from acute pancreatitis. The line labeled "TAP WATER" shows data obtained from a mixture of tap water and the above-described blood, and the line labeled "REDUCE WATER" indicates data obtained from a mixture of cathode water of the present invention and blood. It is appreciated that the amount of SAR does not decrease with tap water. In contrast, there is a significant decrease in the amount of SAR with the cathode water of the present invention.

The pH of tap water was 5.8 ~ 8.6, and the oxidation-reduction potential (ORP) was +200 mV ~ +700 mV.

Mode 1

The values of the pH and oxidation-reduction potential (ORP) of the generated water are shown in Table 1 when electrolyzed (current apply time 0.5 - 5 seconds) under various electrolytic currents (A) (generally expressed by current density ($mA/cm^2$)). The current density is shown per one pair of electrode (two) and diaphragm (one). From these experiments, it was found that the current density is preferably $0.16\ mA/cm^2 \sim 3.2\ mA/cm^2$, particularly $0.224\ mA/cm^2 \sim 1.6\ mA/cm^2$.

Table 1

| Electrolytic Current (A) | Current Density ($mA/cm^2$) | pH | ORP (mV) |
|---|---|---|---|
| 0.3 | 1.0 | 8.9 | -43 |
| 1.0 | 3.2 | 9.7 | -142 |
| 1.5 | 4.9 | 10.1 | -188 |
| 2.0 | 6.5 | 10.4 | -210 |

Mode 2

An experiment of boiling the cathode water obtained as described above was carried out to disinfect bacteria included in the cathode water. It was found that cathode water subjected to boiling does not lose the potency to abolish SAR.

Specifically, two types of electrolytic water having an oxidation-reduction potential of 0 mV and -150 mV were boiled. The boiling state was maintained for five minutes, and then cooled for twenty minutes. The respective measured values of pH and ORP are shown in the following Table 2 and Table 3.

Table 2 shows the data measured at Kochi City using water of Kochi City. Table 3 shows the data measured at Kyoto City using water of Kyoto. It was found that sterile water for medical treatment having an oxidation-reduction measured value within the range of -68 mV ~+55 mV is obtained by boiling the cathode water.

Table 2

| | Before Boiling | After Boiling |
|---|---|---|
| pH | 9.26 | 9.64 |
| ORP | 0mV | -55mV |

Table 2   (continued)

|  | Before Boiling | After Boiling |
|---|---|---|
| pH | 11.2 | 10.7 |
| ORP | -150mV | -33mV |

Table 3

|  | Before Boiling | After Boiling |
|---|---|---|
| pH | 8.7 | 9.0 |
| ORP | -10mV | -21mV |
| pH | 9.8 | 9.9 |
| ORP | -172mV | -68mV |

The cathode water (before and after boiling) obtained in the above-described manner was stable for more than 80 hours. It can be preserved without degradation in its property for about one year if sealed.

Second Embodiment

The second embodiment relates to a dialysis apparatus improved so that cathode water having the above-described property can be used as dialysis liquid.

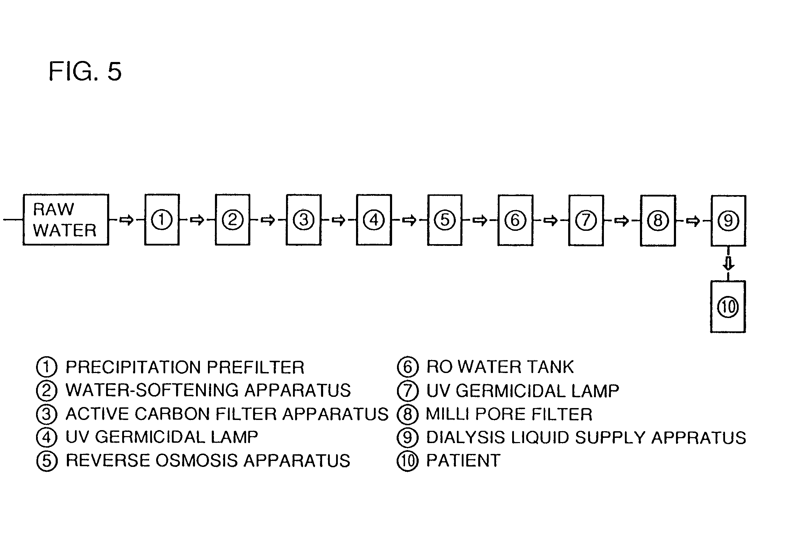

First, a conventional dialysis apparatus will be described with reference to Fig. 5.

Tap water (raw water) is passed through a precipitation prefilter 1 to remove particles. It is fed to a water-softening apparatus 2 to soften the water. Then, chlorin•chloramine•endotoxin is removed by an active carbon filter apparatus 3. The water is then passed through an UV germicidal lamp 4 and then fed to a reverse osmosis apparatus 5. Only approximately 1/2 the water fed into reverse osmosis apparatus 5 under pressurization passes through. The remaining half is processed as drainage. The water passed through is sterile purified water which is then stored in an RO water tank 6. The water is sterilized with an UV germicidal lamp 7 and then conducted through a milli pore filter 8 to be fed into a dialysis liquid supply apparatus 9. Here, the processed water is mixed with undiluted solution of sodium hydrogen carbonate and electrolytic undiluted solution at a predetermined ratio, and passed through a deaerator apparatus to be supplied to a human body 10 as dialysis liquid.

Hemodialysis carried out by a dialysis apparatus is a treatment of substance exchange of the patient's intracorporeal-circulated blood and dialysis liquid (one type of electrolytic liquid) in a dialyzator via a membrane to remove and supply solute in the blood and to remove excessive water. A patient suffering from chronic renal insufficiency is subjected to hemodialysis for approximately 3 times a week, each time taking about 4 hours. Since the flow rate of the dialysis liquid supplied to the dialyzator is normally 500 m$\ell$/minutes, the amount of dialysis liquid used in one treatment is 500 m$\ell$ x 240 minutes = 120,000 m$\ell$ = 120$\ell$ per patient. Since this treatment of hemodialysis requires a great amount of water, the content of the water used in hemodialysis affects the patient greatly. The present embodiment is directed to this fact, and relates to utilizing the above-described cathode water of particular composition in a dialysis apparatus.

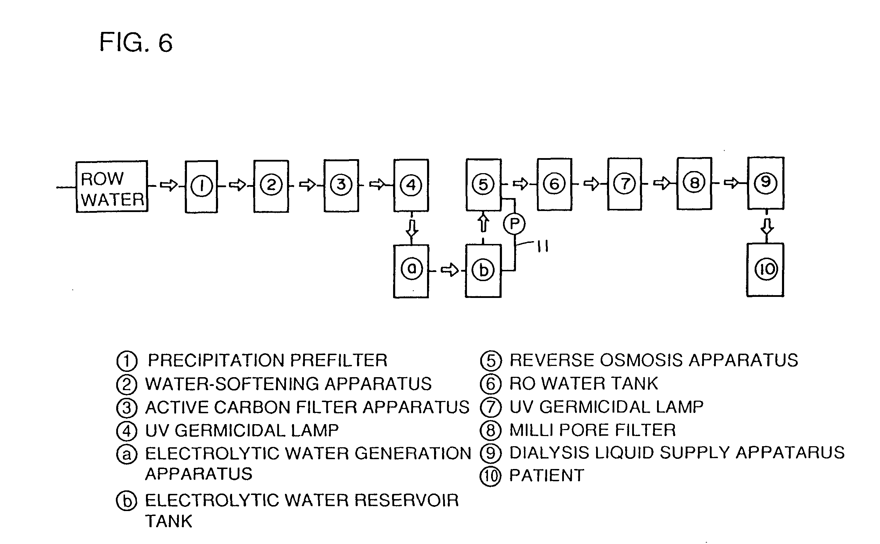

Fig. 6 shows a dialysis apparatus according to the second embodiment of the present invention.

The dialysis apparatus of the second embodiment differs from the conventional dialysis apparatus of Fig. 5 in that an electrolytic water generator apparatus a and an electrolytic water reservoir tank b are provided between UV germicidal lamp 4 and reverse osmosis apparatus 5.

Tap water (raw water) is passed through precipitation prefilter 1 to have particles removed. Next, the water is softened by water-softening apparatus 2 to be supplied to active carbon filtering apparatus 3 to have chlorin•chloramine•endotoxin removed. Then, the water is passed through UV germicidal lamp 4. The sterilized water is fed to electrolytic water generation apparatus a to be electrolyzed. Cathode water obtained by electrolyzation is stored in electrolytic water reservoir tank b and fed to reverse osmosis apparatus 5. Here, half of the fed water passes through, and the remaining water that does not pass through flows through a feedback conduit 11 provided between reverse osmosis apparatus 5 and electrolytic water reservoir tank b to be returned to electrolytic water reservoir tank b by a pump P. The water passed through reverse osmosis apparatus 5 is substantially purified water with no germs. This water is stored in RO water tank 6 and then sent to dialysis liquid supply apparatus 9 via UV germicidal lamp 7 and milli pore filter 8. The processed water is combined with undiluted solution of sodium hydrogen carbonate and electrolytic undiluted solution at a predetermined ratio. The mixture is passed through a deaerator to be supplied to human body 10 as dialysis liquid.

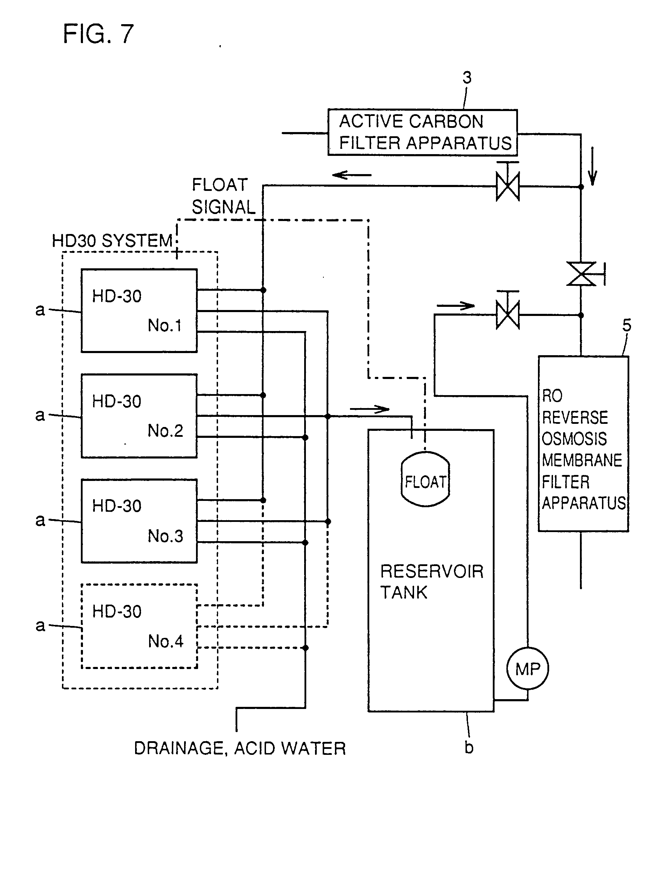

Fig. 7 shows the details of active carbon filter apparatus 3, electrolytic water generation apparatus (HD-30)a, electrolytic water reservoir tank b, and reverse osmosis apparatus 5 shown in Fig. 6.

The method of the present invention is not limited to use of four electrolytic water generators (HD-30) shown in the present embodiment.

Also, the method of the invention is not limited to use of the present dialysis apparatus having an electrolytic liquid reservoir tank provided. An electrolytic water reservoir tank is not necessarily required, and water can be supplied directly to reverse osmosis apparatus 5 from electrolytic water generation apparatus a.

In the present embodiment, a reverse osmosis filtering apparatus is shown as an apparatus for filtering cathode water. Alternatively, cathode water can be filtered with a hollow fiber membrane filter.

Using water produced by the method of the present invention with dialysis apparatus, SAR in the blood of a patient can be removed in addition to the effect of a conventional hemodialysis treatment. The frequency of being subjected to dialysis for a patient suffering from chronic renal insufficiency is reduced to shorten the time required for the treatment. There is an advantage that the time of the patient bound to the dialysis apparatus can be shortened.

Third Embodiment

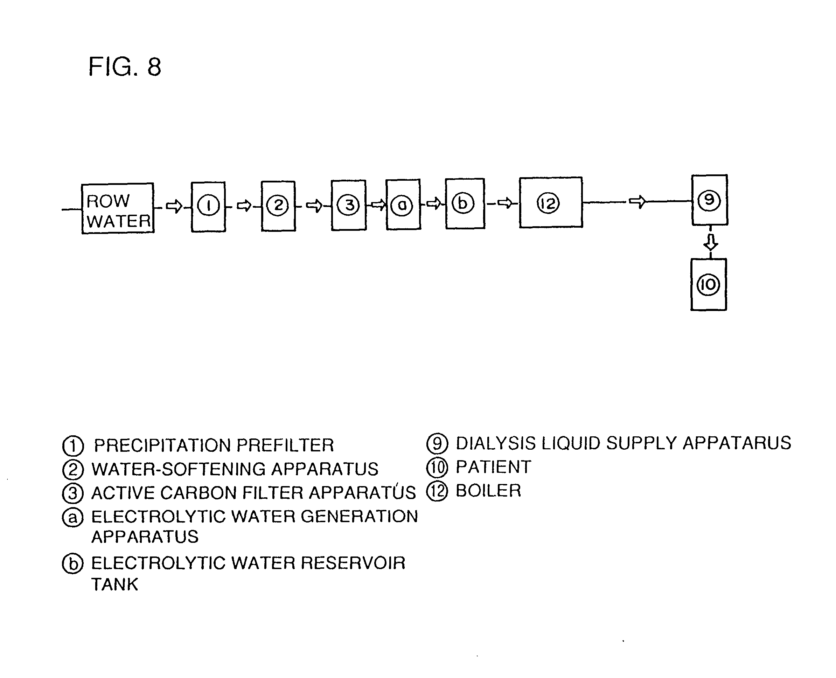

A dialysis apparatus according to a third embodiment shown in Fig. 8 is similar to the dialysis apparatus of Fig. 6 except for the following points. Like or corresponding components have the same reference characters allotted, and their description will not be repeated.

More specifically, the dialysis apparatus according to the third embodiment of the present invention includes a boiler 12 which serves as boiling means, and does not include sterilizing means such as a UV germicidal lamp, a reverse osmosis apparatus, an RO liquid tank, a UV germicidal lamp, and a milli pore filter. Water introduced from electrolytic water reservoir tank b to boiler 12 is boiled and sterilized. Then, the sterile water is provided to dialysis liquid supply apparatus 9 to be applied to patient 10. By substituting sterilization means with such a boiler 12, the cost and burden of maintenance are relieved in contrast to the case where sterilization means such as a UV germicidal lamp is used.

The present invention is not limited to the above-described first to third embodiments in which the water for medical treatment of the above-described property is used for cleaning blood, and can be used as Ringer's solution and cleaning water used for operation.

## Claims

1. A method of producing cathode water having an oxidation-reduction potential value within a range of -150 mV ~ 0 mV measured against a platinum electrode, comprising the steps of:

   providing an electrolytic water treatment apparatus including a cathode chamber with a cathode and an anode chamber with an anode, said chambers separated by a diaphragm;

   introducing raw water including at least sodium, potassium, magnesium, and calcium ions into said cathode chamber and said anode chamber;

   applying a current within a range of $0.16 \text{ mA/cm}^2 \sim 3.2 \text{ mA/cm}^2$ per each pair of electrodes and one diaphragm across said cathode and anode for 0.5 seconds to 5 seconds for electrolyzing said raw water; and

   extracting electrolyzed water from said cathode chamber.

2. The method according to claim 1, wherein said current is within a range of $0.224 \text{ mA/cm}^2 \sim 1.6 \text{ mA/cm}^2$.

3. The method according to claim 1, further comprising the step of boiling said extracted electrolyzed water.

## Patentansprüche

1. Verfahren zum Erzeugen von Kathodenwasser mit einem Oxidations-Reduktions-Potenzialwert in einem Bereich von -150 mV ~ 0 mV gemessen an einer Platinelektrode, das die Schritte umfasst:

   Vorsehen einer Behandlungsvorrichtung für elektrolytisches Wasser, die eine Kathodenkammer mit einer Kathode und eine Anodenkammer mit einer Anode aufweist, wobei diese Kammern durch eine Membran getrennt sind;

Einführen von Frischwasser, das wenigstens Natrium-, Kalium-, Magnesium- und Calciumionen aufweist, in die Kathodenkammer und die Anodenkammer;

Anlegen eines Stroms in einem Bereich von 0,16 mA/cm$^2$ $\sim$ 3,2 mA/cm$^2$ für jedes Paar Elektroden und eine Membran über die Kathode und die Anode für 0,5 Sekunden bis 5 Sekunden zur Elektrolysierung des Frischwassers; und

Abziehen des elektrolysierten Wassers aus der Kathodenkammer.

2. Verfahren nach Anspruch 1, bei dem der Strom in einem Bereich von 0,224 mA/cm$^2$ $\sim$ 1,6 mA/cm$^2$ liegt.

3. Verfahren nach Anspruch 1, das weiterhin den Schritt des Kochens des abgezogenen elektrolysierten Wassers umfasst.

**Revendications**

1. Méthode de production d'eau de cathode ayant une valeur de potentiel d'oxydo-réduction comprise dans un intervalle de - 150 mV - 0 mV mesurée par rapport à une électrode en platine, comprenant les étapes de :

- fourniture d'un appareil de traitement électrolytique de l'eau incluant une chambre de cathode avec une cathode et une chambre d'anode avec une anode, lesdites chambres étant séparées par un diaphragme ;
- introduction d'eau brute incluant au moins des ions sodium, potassium, magnésium et calcium dans ladite chambre de cathode et ladite chambre d'anode ;
- application d'un courant compris dans un intervalle de 0.16 mA/cm$^2$ $\sim$ 3.2 mA/cm$^2$ pour chaque paire d'électrodes et d'un diaphragme à travers lesdites cathode et anode pendant 0.5 secondes à 5 secondes pour électrolyser ladite eau brute ; et
- extraction de l'eau électrolysée à partir de ladite chambre de cathode.

2. Méthode selon la revendication 1, dans laquelle ledit courant est compris dans un intervalle de 0.224 mA/cm$^2$ $\sim$ 1.6 mA/cm$^2$.

3. Méthode selon la revendication 1, comprenant en outre l'étape de faire bouillir ladite eau électrolysée extraite.

# FIG. 1

## FIG. 2

AGE:72  SEX:M

| NO. | FILE | SAMPLE | TIMES | GATE [sec] | TEMP. [C] | TOTAL COUNTS | DARK AV. |
|---|---|---|---|---|---|---|---|
|  | A1-6 | 3180932-1 | 60 | 10 | 36 | 337906 | 569.1 |
| 99 | A1-7 | 3180932-1 | 60 | 10 | 36 | 90734 | 570.2 |
| 100 | A1-82 | 3180932-1 | 60 | 10 | 36 | 87637 | 589.3 |
| 97 | A1-9 | 3180932-1 | 60 | 10 | 36 | 52522 | 555.5 |
| 98 | A1-10 | 3180932-1 | 60 | 10 | 36 | 86037 | 569.0 |

| ml | | COUNTS/10secs | % | ORP (mv) | PH | TEMP. ℃ | A |
|---|---|---|---|---|---|---|---|
| PBS | 317521 | /40-569.1=7368.93 | | | 8.5 | 21 | |
| 1 | 78018 | /40-570.2=1380.25 | ↓ 81.3 | -33 | 8.15 | 21.7 | 0.75 |
| 2 | 75286 | /40-589.3=1292.85 | ↓ 82.5 | -59 | 9.06 | 21.5 | 1 |
| 3 | 40873 | /40-555.5= 466.33 | ↓ 93.7 | -99 | 9.24 | 21.1 | 1.5 |
| 4 | 73287 | /40-569.0=1263.18 | ↓ 82.9 | -111 | 9.40 | 21.1 | 2 |

CL INTENSITY

CONTROL

2°
4°
1°

3°

TIME (sec)

9

## FIG. 3

AGE:38 SEX:M

| NO. | FILE | SAMPLE | TIMES | GATE [sec] | TEMP. [C] | TOTAL COUNTS | DARK AV. |
|---|---|---|---|---|---|---|---|
| 129 | TH-2 | TH-LIAOM38 | 60 | 10 | 37 | 134602 | 587.8 |
| 130 | TH-RW1 | TH-LIAOM38 | 60 | 10 | 37 | 67416 | 578.1 |
| 131 | TH-3 | TH-LIAOM38 | 60 | 10 | 37 | 132030 | 591.7 |

| ml | COUNTS/10secs | | % | ORP (mv) | PH | TEMP. °C | A |
|---|---|---|---|---|---|---|---|
| PBS | 117806 | /40-587.8=2357.4 | | | | | |
| 1 | 55116 | /40-578.1= 799.8 | ↓ 66.1 | -52 | 7.45 | | 0.5 |
| 2 | | /40- = | | | | | |
| 3 | | /40- = | | | | | |
| 4 | 118931 | /40-591.7=2376.5 | ↑ 0.8 | -204 | 9.95 | | 3 |

CL INTENSITY

TIME (sec)

CONTROL

4°

1°

FIG. 4

FIG. 5

① PRECIPITATION PREFILTER       ⑥ RO WATER TANK
② WATER-SOFTENING APPARATUS       ⑦ UV GERMICIDAL LAMP
③ ACTIVE CARBON FILTER APPARATUS   ⑧ MILLI PORE FILTER
④ UV GERMICIDAL LAMP       ⑨ DIALYSIS LIQUID SUPPLY APPRATUS
⑤ REVERSE OSMOSIS APPARATUS       ⑩ PATIENT

# FIG. 6

① PRECIPITATION PREFILTER    ⑤ REVERSE OSMOSIS APPARATUS
② WATER-SOFTENING APPARATUS    ⑥ RO WATER TANK
③ ACTIVE CARBON FILTER APPARATUS ⑦ UV GERMICIDAL LAMP
④ UV GERMICIDAL LAMP    ⑧ MILLI PORE FILTER
ⓐ ELECTROLYTIC WATER GENERATION ⑨ DIALYSIS LIQUID SUPPLY APPATARUS
APPARATUS    ⑩ PATIENT
ⓑ ELECTROLYTIC WATER RESERVOIR
TANK

EP 0 752 391 B1

FIG. 7

# FIG. 8

① PRECIPITATION PREFILTER
② WATER-SOFTENING APPARATUS
③ ACTIVE CARBON FILTER APPARATUS
ⓐ ELECTROLYTIC WATER GENERATION
 APPARATUS
ⓑ ELECTROLYTIC WATER RESERVOIR
 TANK

⑨ DIALYSIS LIQUID SUPPLY APPATARUS
⑩ PATIENT
⑫ BOILER

EP 0 752 391 B1